# EUROPEAN PATENT APPLICATION

(11) **EP 4 751 699 A1**
(43) Date of publication of application: **03.06.2026**
(21) Application number: 24216014.1
(22) Date of filing: 28.11.2024
(51) Int. Cl.: A61K 8/02, A61K 8/25, A61Q 11/00

(54) **COMPOSITION**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

A film forming oral care composition used to treat the surface of at least one tooth wherein the composition comprises; a tubule blocking agent comprising a bioactive glass, wherein the tubule blocking agent has a particle size less than 10 µm, and wherein the composition is a leave on composition, and wherein the composition is free of one or more of, an abrasive, an opacifier, a fluoride, and / or surfactant;

## Description

### BACKGROUND

The present invention relates to a composition and a method of using a composition for tooth remineralisation and/or desensitization. More particularly, the present invention relates to a composition comprising a bioactive glass and a water soluble polymer and a method of using such a composition for tooth remineralisation and/or desensitization.

### BACKGROUND OF THE INVENTION

It is well known that tooth structure comprises an inner dentine layer and an outer hard enamel layer that serves to protect the tooth. The enamel layer is composed of dense calcium hydroxyapatite mineral crystals; in the form of large "prismatic enamel" crystals surrounded by smaller crystals that fill in the "inter-prismatic" spaces.

The formula of calcium hydroxy apatite is nominally Ca₅(PO₄)₃OH. The dentine however is composed of both hydroxy apatite, as well as structural proteins that result in a much softer and less dense structure than enamel and which is much easier to damage. The dentine structure also consists of pores or "tubules" which create openings from the tooth surface that run down into the pulp cavity of the tooth where the tooth nerve is located.

Normally this softer porous dentine structure is covered by the denser enamel layer, or by the soft gingival tissue, which protects the nerve from outside stimuli. Tooth hypersensitivity is associated with the exposure of these tubules, through loss of the protective enamel layer or recession of the gums, that tends to increase with age.

The recession of the gums can also be caused by overbrushing, as well as periodontal disease. The loss of enamel can occur as a result of acid erosion from acidic foods or cariogenic bacteria and abrasion from over brushing or other mechanical effects. Orthodontic brace wearers are also especially susceptible to loss of tooth enamel, leading to white spot lesions.

Demineralisation of tooth enamel tends to reveal tubules. Dentinal tubules are naturally present in the enamel and they provide for an osmotic flow of liquid between the inner pulp region of the tooth and the outer root surfaces. However, when demineralisation of the enamel layer occurs, these tubules tend to become exposed. When heat, cold, or pressure are applied, fluids tend to gain direct access to the tooth nerve causing pain. It is possible to alleviate this condition by coating the teeth with varnishes, lacquers etc., but this tends to be a short term remedy, since the lacquers become quickly worn away.

Bioactive glasses, alone or incorporated into oral care compositions, are known to remineralise teeth. When applied to the teeth they have the effect of rebuilding the hydroxy apatite structure. For example, US Patents 6,338,751 and 6,086,374, relate to oral care products which comprise remineralisation compositions (typically comprising calcium, phosphorous, silica and sodium ions in the form of a bioactive glass). The bioactive glass incorporated into these products releases remineralising ions onto the tooth's surface and helps occlude the open tubules, reducing tooth sensitivity, and rebuilding the enamel layer. However, a disadvantage of these compositions is that the contact time for toothpastes or dentifrices is limited to the time of brushing and so may only be a minute or two.

Accordingly, there is a desire for oral care compositions, that are alleviating dentinal (hyper) sensitivity for a user.

### STATEMENT OF INVENTION

A film forming oral care composition used to treat the surface of at least one tooth wherein the composition comprises;
a tubule blocking agent comprising a bioactive glass, wherein the tubule blocking agent has a particle size less than 10 µm
and wherein the composition is a leave on composition.
and wherein the composition is free of one or more of:-
   abrasive,
   opacifier,
   fluoride, and / or
   surfactant;

The invention may also comprise the use of the composition to clean teeth. Ideally the composition is applied manually (i.e. not with a toothbrush). Preferred forms of manual application include manual application using a finger or thumb to rub the composition onto a tooth or gum surface. Once applicated in this way further spreading may be aided by lingual movement and / or movement of saliva within the mouth.

Preferably, however, the composition is a "leave-on" composition. As such it is intended for being left on teeth for (as an example) at least 4 hours. Ideally it is intended to be left on teeth overnight.

By this it is intended that a user applies the composition to a tooth surface at, or just before, the user retires to sleep. Sleep is understood to usually occur (for the longest period) at night; although it is appreciated that this is not always the case. Here of course it is realised that there is no fixed time in which users retire to sleep and that this is influenced by a number of factors including age and responsibilities (such as employment). However, it is understood that retirement to sleep will generally be understood to comprise a period of approximately about 4 hours to about 10 hours; and may be longer or shorter depending on an individual user's circumstances. In this way the composition is able to act on the tooth surface whilst the user sleeps.

The composition may then be (at least partially) actively or passively removed when the user awakens. This removal may be in a teeth cleaning operation. Alternatively / additionally, the removal may occur by eating and / or drinking, such as at breakfast.

Without wishing to be bound by theory it is postulated that the composition of the present invention is capable of forming a (thin) film round a surface (such as a dental surface) to which it is applied. As such, generally speaking, the present invention may reside in the provision of a remineralisation composition comprising a bioactive glass in the form of a thin film, which may be adhered to at least one tooth, and provides a convenient and unobtrusive method of generating a high and sustained local concentration of remineralising and/or desensitizing components on a tooth surface.

The composition is free of at least one or more of abrasives, opacifiers, fluoride and surfactants.

Preferably the composition is free of abrasive. Preferably the composition is free of opacifier. Preferably the composition is free of fluoride. Preferably the composition is free of surfactant.

Most preferably the composition is free of abrasive, opacifier, fluoride, and surfactant.

As used herein the term "free of" means less than 0.1 % by weight, and preferably less than 0.05% by weight of the component.

As used herein the term "oral care composition" includes any composition for treating all, or a portion of, the oral cavity of an individual.

As used herein the term "dentifrice" includes any semi-solid preparation in the form of a paste, cream or gel for use in cleaning or treating all, or a portion of, the oral cavity of an individual.

The present inventors have discovered that the dentifrice of the invention provides excellent sensitivity protection; allowing users to whiten teeth/dentures with confidence.

In more detail it has been found that the dentifrice of the invention, by reducing the particle size of the tubule blocking agent increased the reactivity of same. This is associated with greater reactivity and faster apatite formation (in, for example, the first 24 hours).

It had no significant effect on the mechanical or crystallographic properties of the HA-like surface layer formed by the dentifrice (even after, for example, 5 days of development). This has been found to be associated with no change in hardness or reduced modulus and no change in crystallite size.

It has been found to result in greater tubule occlusion and greater penetration of material deeper into the tubules (even after, for example, only 24 and 48 hours).

### DETAILED DESCRIPTION OF THE INVENTION

### Tubule Blocking Agent Particle Size

The tubule blocking agent preferably has a particle size of less than about 10 µm.

Preferably the tubule blocking agent has a particle size less than about 9 µm, more preferably less than about 8 µm, more preferably less than about than 7 µm, more preferably less than about 6 µm, more preferably less than about 5 µm, more preferably less than about 4 µm, more preferably less than about 3 µm, more preferably less than about 2.5 µm, more preferably less than about 2.25 µm, more preferably less than about 2.1 µm, more preferably less than about 2 µm.

Alternatively and / or additionally the tubule blocking agent preferably has an average particle size in the range of about 0.01 to about 10 µm, more preferably in the range of about 0.01 to about 9 µm, more preferably in the range of about 0.01 to about 8 µm, more preferably in the range of about 0.01 to about 7 µm, more preferably in the range of about 0.01 to about 6 µm, more preferably in the range of about 0.01 to about 5 µm, more preferably in the range of about 0.01 to about 4 µm, more preferably in the range of about 0.01 to about 3 µm, more preferably in the range of about 0.01 to about 2.5 µm, more preferably in the range of about 0.01 to about 2.25 µm, more preferably in the range of about 0.01 to about 2.1 µm, more preferably in the range of about 0.01 to about 2 µm. In each of these ranges the lower amount (about 0.01 µm) may be replaced by about 0.1 µm, about 0.2 µm, about 0.3 µm, about 0.4 µm, about 0.5 µm, about 0.6 µm, about 0.7 µm, about 0.8 µm, about 0.9 µm, about 1.0 µm, 1.1 µm, about 1.2 µm, about 1.3 µm, about 1.4 µm, about 1.5 µm, about 1.6 µm, about 1.7 µm, about 1.8 µm, or about 1.9 µm (wherein the tubule blocking agent has a particle size less than 10 µm).

Alternatively and / or additionally the tubule blocking agent preferably has a particle size in the range of about 0.01 to about 10 µm, more preferably in the range of about 0.01 to about 9 µm, more preferably in the range of about 0.01 to about 8 µm, more preferably in the range of about 0.01 to about 7 µm, more preferably in the range of about 0.01 to about 6 µm, more preferably in the range of about 0.01 to about 5 µm, more preferably in the range of about 0.01 to about 4 µm, more preferably in the range of about 0.01 to about 3 µm, more preferably in the range of about 0.01 to about 2.5 µm, more preferably in the range of about 0.01 to about 2.25 µm, more preferably in the range of about 0.01 to about 2.1 µm, more preferably in the range of about 0.01 to about 2 µm. In each of these ranges the lower amount (about 0.01 µm) may be replaced by about 0.1 µm, about 0.2 µm, about 0.3 µm, about 0.4 µm, about 0.5 µm, about 0.6 µm, about 0.7 µm, about 0.8 µm, about 0.9 µm, about 1.0 µm, 1.1 µm, about 1.2 µm, about 1.3 µm, about 1.4 µm, about 1.5 µm, about 1.6 µm, about 1.7 µm, about 1.8 µm, or about 1.9 µm.

Particle size measurements carried out by laser diffraction according to standard techniques.

### Amount of Tubule Blocking Agent

Preferably the tubule blocking agent is present in an amount of 0.1 to 20 percent by weight of the composition. The tubule blocking agent may be present in an amount of 1 to 19 percent, 2 to 18 percent, 3 to 17 percent, 4 to 16 percent, about 5 to about 15 percent, by weight of the composition.

### Nature of Tubule Blocking Agent

The tubule blocking agent includes a bioactive glass.

A suitable example of a bioactive glass consists of 45 percent by weight silicon dioxide, 24.5 percent by weight sodium oxide, 6 percent by weight phosphorus oxide, and 24.5 percent by weight calcium oxide. (45 wt% SiO₂, 24.5 wt% CaO, 24.5 wt% Na₂O, and 6.0 wt% P₂O₅₎ This bioactive glass is available commercially under the trade name, NOVAMIN, also known as 45S5 BIOGLASS.

Another alternate tubule blocking agent may be present in addition to the bioactive glass.

The alternate tubule blocking agent is preferably selected from the list comprising silica, colloidal silica, nano zinc oxide, sub-micron alumina and sub-micron polymer beads, or mixtures thereof.

Where present the alternate tubule blocking agent preferably has a similar size to the bioactive glass.

### Anti-sensitivity

An additional desensitizing agent, including a tubule blocking agent or a nerve desensitizing agent and mixtures thereof, for example as described in WO 02/15809 (Block) is included in a composition according to the invention.

Preferably the additional desensitizing agent is present in an amount of 0.1 and 5 percent by weight of the composition.

The desensitizing agent may comprise a strontium salt such as strontium chloride, strontium acetate or strontium nitrate or a potassium salt such as potassium citrate, potassium chloride, potassium bicarbonate, potassium gluconate and especially potassium nitrate.

### Diluent

Preferably the dentifrice comprises a single phase non-aqueous composition comprising at least one solvent.

The diluent preferably provides a film-forming ability (see above)

The at least one solvent comprises glycerol, polyethylene glycol (PEG) or a mixture thereof.

Glycerine is also known as glycerol and glycerin. The terms may be used interchangeably in this specification.

It is well known that commercially available glycerine may contain between 0.1-2.0 percent by weight of water which is in association with the glycerine. Typically, this amount is < 0.5 percent and for example between 0.1 - 0.5 percent by weight of the glycerine. This small amount of water is bound to the glycerine and is therefore not available to the other ingredients. The skilled person would still consider a composition containing glycerine as being non-aqueous.

Preferably the at least one solvent comprises up to 95 percent by weight of the final composition. Preferably the amount of glycerol is up to 80 percent by weight of the final composition; most preferably about 75 percent by weight. Preferably the amount of polyethylene glycol (PEG) is up to 25 percent by weight of the final composition; most preferably about 20 percent by weight.

### Gel

The non-aqueous dentifrice composition according to the invention comprise a supporting (or gelling) polymer to solidify the solvent and actives.

The skilled person will be aware of many different types of supporting polymer for dentifrice use. Two non-limiting examples include a copolymer of a methyl vinyl ether and a maleic anhydride, such as Gantrez(R), and polyacrylic acid homopolymers, often referred to as Carbomers.

Suitably for the present invention the supporting (or gelling) polymer comprises a carboxyvinyl polymer such as a carbomer.

A carbomer comprises synthetic high molecular-weight cross-linked polymers of acrylic acid. The polymer chains formed of repeating units of acrylic acid may be cross-linked with, for example: allyl sucrose to provide a carbomer available commercially in one form as Carbopol^{™} 934; ethers of pentaerythritol to provide a carbomer available commercially in one form as Carbopol^{™} 974; or with divinyl glycol, available commercially in one form as Noveon^{™} AA-1. Carbopol^{™} polymers are manufactured by B.F. Goodrich Company.

In one embodiment the carboxyvinyl polymer comprises Carbopol^{™} 974.

The carboxyvinyl polymer may be present in the range of from about 0.1 to about 7.5 percent by weight of the non-aqueous composition. In one embodiment the carboxyvinyl polymer is present in an amount from about 0.2 to about 1.0 percent by weight of the composition.

### Abrasive

The dentifrice compositions of the present invention are preferably free of abrasive.

Compositions of the present invention are preferably not used to clean teeth and therefore would not need an abrasive component.

However, an abrasive can be added if required. Any abrasive known for use in oral care compositions may be used in conjunction with the composition of the present invention.

Abrasives are common ingredients in regular toothpastes. An abrasive is usually required to aid the removal of detritus from the teeth, dentures and / or remainder of the oral cavity being cleaned. Clearly it will be appreciated that a degree of abrasiveness is required / advantageous in the removal of said detritus., However, it will also be appreciated that excessive abrasiveness can be equally detrimental: excessive wear on dental materials can be problematic. As such it will be appreciated that the use of an abrasive is a compromise of achieving adequate detritus removal without causing excessive /undesired wear on dental materials.

Suitable abrasives for use in dentifrice compositions include, for example, amorphous, gelled, precipitated or fumed silica, zinc orthophosphate, sodium bicarbonate (baking soda), plastic particles, alumina, hydrated alumina, calcium carbonate, calcium pyrophosphate, insoluble metaphosphates or mixtures thereof.

The silica abrasive may be a natural amorphous silica, for instance diatomaceous earth; or a synthetic amorphous silica such as a precipitated silica. By way of example, silica abrasives include those marketed under the following trade names Zeodent, Sident, Sorbosil or Tixosil by Huber, Degussa, Ineos and Rhodia respectively.

The silica abrasive may be a spherical, anhydrous, amorphous, silica gel particles having a pore volume of less than 0.1ml/g (as described in co-pending European Application Number 18782931.2). This abrasive preferably comprises non-fused spherical, anhydrous, amorphous, silica gel particles obtainable by a process that involves precipitation followed by calcination to remove water to produce a dense particulate silica material, and wherein said silica gel particles comprise:
a. a pore volume from 0.03 ml/g to less than 0.1 ml/g;
b. a mean particle size from 1 micro to 10 micro;
c. a BET surface area of 50 m²/g or less;
d. an oil absorption capacity of 20 to 50 ml/100 g; and
e. a water content of less than 0.2 weight percent.

Such silica gel particles have been found to effectively clean, polish and remove stains from the surface of teeth or dentures without a high degree of abrasion thereby reducing scratching and damage to the tooth or denture surface. Such compositions thereby provide superior cleaning, polishing, gentle stain removal and whitening of tooth surfaces or dentures.

Silica gel particles for use in the invention are available commercially from Asahi Glass SI-Tech. Co., Ltd., 13-1, Kitaminatomachi,,Wakamatsu-ku, Fukuoka, 808-0027, Japan. and are sold under the brand name Sunspherc(a), for example Sunsphcre NP-30 and Sunsphcrc NP-100. A CAS¹ for some types of silica gel particles of use in the invention is 7631-86-9.

Sunsphere(a) silica gel particles are derived from sodium silicate and contain no crystalline silica. They consist of spherical particles of almost identical size which do not cohere, and which provide a smooth texture.

Similar silica gel particles are available from Madhu Silica PVT. LTD under the brand name ( as an example) of MFIL-GS.

Suitably an abrasive is present in an amount up to 5 percent by weight of the total composition, for example from 0.1 to 4 percent by weight, for example from 0.1 to 3 percent, for example from 0.1 to 2 percent, for example from 0.1 to 1.5 percent, for example from 0.2 to 1 percent, for example from 0.25 to 1 percent by weight of the total composition.

Generally, an amount of abrasive suitable for use in dentifrice composition of the present invention will be empirically determined to provide an acceptable level of cleaning and polishing, in accordance with the techniques well known in the art.

### Fluoride

The dentifrice compositions of the present invention are preferably free of fluoride.

By this it is meant that the compositions comprise less than 0.05wt% of a fluoride source.

### Thickening agent

The compositions of the present invention may comprise a thickening agent to achieve a desirable viscosity. Any thickening agent known in the art for oral care compositions may be used.

Anon-limiting example is thickening silica. This has little or no abrasive characteristics.

Suitable examples of a thickening silica are available commercially for example as Sident 22S or Syloid 244FP.

In one embodiment the thickening silica is present in the range of from about 0 to about 5.0 % by weight of the non-aqueous composition.

### Surfactant

The dentifrice compositions of the present invention are preferably free of surfactant.

By this it is meant that the compositions comprise less than 0.05 wt% of a surfactant source.

### Other

Compositions of the present invention may contain additional formulating agents such as flavouring agents, sweetening agents, opacifying or colouring agents and preservatives, selected from those conventionally used in an oral hygiene composition art for such purposes.

In general, the optional agents may be used in a minor amount or proportion of the overall formulation. By way of example, such components are usually present in from about 0.001 to about 5 percent by weight of the non-aqueous composition. In total such components may range from 0.1 to 25 percent by weight of the composition.

The dentifrice composition typically has a viscosity suitable for application to the oral cavity. The viscosity will vary depending on the type of dentifrice composition made and the ultimate use thereof. One of skill in the art can readily prepare compositions with suitable viscosities for use in the oral cavity from the teachings provided herein.

The compositions according to the present invention may be prepared by admixing the ingredients in the appropriate relative amounts in any order that is convenient.

A dentifrice composition according to the present invention may be mildly acidic, neutral or mildly alkaline i.e. has a slurry pH in the range from 6.0 to 7.5, for example from pH 6.1 to 7.4, 6.2 to 7.3, or 6.2 to 7.2. In one embodiment the composition has a pH of about 6.2. In a further embodiment the composition has a pH of about 7.0. The pH referred to is that measured when the dentifrice composition is slurried with water in a 1:3 weight ratio of the composition to water. Suitably the slurry is prepared by slurring the dentifrice composition with water in a weight ratio of one part dentifrice composition and three parts distilled water. The pH is determined using a standard pH meter.

### Examples

The following formulae representing the invention were prepared:-

| **Component** | **Formula (all amounts shown as wt.%)** | | | | | |
|---|---|---|---|---|---|---|
| | **1** | **2** | **3** | **4** | **5** | **6** |
| Glycerol | 70.5 | 69.6 | 71.2 | 63.2 | 72.9 | 74.9 |
| polyethylene glycol (PEG) (Macrogol 400) | 20 | 20 | 20 | 20 | 20 | 20 |
| Thickening agent (silica) | 1.0 | 2.0 | 2.7 | 0.0 | 1.0 | 4.0 |
| Cross-linked polymers of acrylic acid (Carbopol 974P) | 0.84 | 0.84 | 0.84 | 0.84 | 0.84 | 0.84 |
| Sweetener | 0.05 | 0.05 | 0.05 | 0.05 | 0.05 | 0.05 |
| Bioactive glass (NOVAMIN, 45S5) | 7.5 | 7.5 | 5.0 | 15.0 | 5.0 | 0.0 |
| Flavouring | 0.2 | 0.2 | 0.2 | 0.2 | 0.2 | 0.2 |
| Pigment | 0.0002 | 0.0002 | 0.0002 | 0.0002 | 0.0 | 0.0002 |

### Results

All of the formulas above demonstrated successful tubule occlusion when applied to teeth in in vitro testing.

## Claims

1. A film forming oral care composition suitable to treat the surface of at least one tooth, wherein the composition comprises;
a tubule blocking agent comprising a bioactive glass, wherein the tubule blocking agent has a particle size less than 10 µm
and wherein the composition is a leave on composition.
and wherein the composition is free of one or more of:-
an abrasive,
an opacifier,
a fluoride, and / or
a surfactant;

2. The composition according to claim 1, wherein the tubule blocking agent has a particle size less than about 9 µm, more preferably less than about 8 µm, more preferably less than about than 7 µm, more preferably less than about 6 µm, more preferably less than about 5 µm, more preferably less than about 4 µm, more preferably less than about 3 µm, more preferably less than about 2.5 µm, more preferably less than about 2.25 µm, more preferably less than about 2.1 µm, more preferably less than about 2 µm.

3. The composition according to claim 1, wherein the tubule blocking agent has a particle size in the range of about 0.01 to about 10 µm, more preferably in the range of about 0.01 to about 9 µm, more preferably in the range of about 0.01 to about 8 µm, more preferably in the range of about 0.01 to about 7 µm, more preferably in the range of about 0.01 to about 6 µm, more preferably in the range of about 0.01 to about 5 µm, more preferably in the range of about 0.01 to about 4 µm, more preferably in the range of about 0.01 to about 3 µm, more preferably in the range of about 0.01 to about 2.5 µm, more preferably in the range of about 0.01 to about 2.25 µm. more preferably in the range of about 0.01 to about 2.1 µm, more preferably in the range of about 0.01 to about 2 µm.

4. The composition according to claim 3, wherein in each of these ranges the lower amount (about 0.01 µm) may be replaced by about 0.1 µm, about 0.2 µm, about 0.3 µm, about 0.4 µm, about 0.5 µm, about 0.6 µm, about 0.7 µm, about 0.8 µm, about 0.9 µm, about 1.0 µm, 1.1 µm, about 1.2 µm, about 1.3 µm, about 1.4 µm, about 1.5 µm, about 1.6 µm, about 1.7 µm, about 1.8 µm, or about 1.9 µm.

5. The composition according to any of the preceding claims, wherein the tubule blocking agent is present in an amount of 0.1 to 20 percent by weight of the composition.

6. The composition according to any of the preceding claims, wherein the composition comprises a single phase non-aqueous composition comprising at least one solvent.

7. The composition according to claim 6, wherein the at least one solvent comprises up to 85 percent by weight of the final composition.

8. The composition according to claim 6 or 7, wherein the at least one solvent comprises glycerol, polyethylene glycol (PEG) or a mixture thereof.

9. The composition according to any of the preceding claims, wherein the composition is free of abrasive.

10. The composition according to any of the preceding claims, wherein the composition is free of opacifier.

11. The composition according to any of the preceding claims, wherein the composition is free of fluoride.

12. The composition according to any of the preceding claims, wherein the composition is free of surfactant.

13. A use of the composition according to any of claims 1 to 12 to treat at least one tooth, preferably wherein said composition is applied manually to the at least one tooth surface with a brush or finger.

14. The use according to claim 13, wherein the composition is left on the surface of the at least one tooth for (as an example) at least 4 hours.

15. The use according to claim 13 or 14 wherein the composition is left on the surface of the at least one tooth overnight.
